(19) Europäisches Patentamt
European Patent Office
Office européen des brevets

(11) Publication number : 0 528 629 A2

(12) # EUROPEAN PATENT APPLICATION

(21) Application number : **92307368.8**

(22) Date of filing : **12.08.92**

(51) Int. Cl.$^5$ : **C07K 5/06,** C12P 21/02

(30) Priority : **12.08.91 US 744077**

(43) Date of publication of application : **24.02.93 Bulletin 93/08**

(84) Designated Contracting States : **AT BE CH DE DK ES FR GB GR IE IT LI LU MC NL PT SE**

(71) Applicant : **BEND RESEARCH, INC.**
**64550 Research Road**
**Bend Oregon, 97701 (US)**

(72) Inventor : **Van Eikeren, Paul**
**1922 N.W. Seventh Street**
**Bend, Oregon 97701 (US)**
Inventor : **Blair, West J.**
**63278 Whitewing Court**
**Bend, Oregon 97701 (US)**

(74) Representative : **Skerrett, John Norton Haigh et al**
**H.N. & W.S. SKERRETT Charles House 148/9 Great Charles Street**
**Birmingham B3 3HT (GB)**

(54) **Enzymatic synthesis of renin inhibitors.**

(57) Peptide renin inhibitors are synthesized by coupling component peptide acyl donors and nucleophiles in the presence of a class of proteases exhibiting certain minimum reaction velocities, regioselectivities and diastereoselectivities in a standardized enzymatic coupling reaction. An especially preferred subclass of proteases constitutes sulfhydryl proteases.

EP 0 528 629 A2

Background of the Invention

Renin inhibitors of the structure

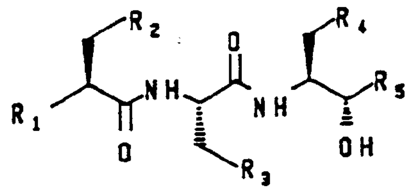

are a known class of anti-hypertensive agents that are the subject of increasing interest as cardiovascular drugs. See Greenlee, "Renin inhibitors," 4 Pharm. Res. 364 (1987). Because of this, there has been considerable interest in the development of efficient methods of synthesizing certain classes of peptides exhibiting renin-inhibition properties, as well as intermediates thereof.

Such agents of the above general structure are conventionally prepared by the reaction of amino acid or peptide derivatives (peptide acyl donors) of the general structure I (where R is hydrogen) with β-hydroxy amine derivatives (nucleophiles) of the general structure II in the presence of chemical coupling reagents to form a peptide bond according to the reaction scheme shown below:

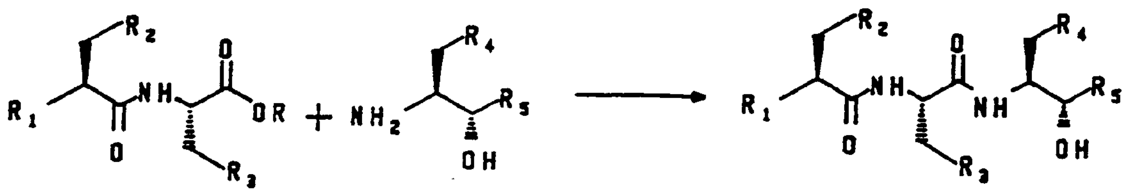

I II III

See, for example, European patent applications EP312157A2, EP309841A2, and EP310070A2. Such chemical methods, however, involve the risk of undesirable secondary reactions and racemizations, and it is therefore necessary to control the chemical reactions carefully to minimize or eliminate these problems. An especially undesirable secondary reaction is the incorporation of unwanted D-amino acid-containing peptide acyl donors, which leads to diastereomeric impurities, rendering the renin inhibitor product useless for its intended purpose. In addition, the reaction speed and yield of such methods is often low, and secondary reactions necessitate cumbersome purification procedures to obtain pure products. Furthermore, such chemical methods often employ toxic solvents, thereby creating waste disposal problems. Such inherent problems of chemical syntheses and the high cost of the coupling reagents make such renin inhibitors relatively expensive to produce.

Enzymes are known to be highly specific catalysts that operate in aqueous solution at room temperature. Since protease enzymes are known to catalyze the hydrolysis of peptides, much effort has been directed at exploring the practical feasibility of reversing the hydrolysis reaction--that is, to use proteases to catalyze the synthesis of peptides. The use of protease enzyme catalysts to couple α-amino acids or α-amino acid derivatives to other α-amino acids or α-amino acid derivatives is well known in the art. See, for example, U.S. Patent No. 4,806,473.

The use of protease enzyme catalysts to couple α-amino acids or α-amino acid derivatives to non-α-amino acid amines or amine derivatives is less well known. Enzyme-catalyzed coupling can be achieved by kinetic methods (a nonequilibrium approach that requires directing the partitioning of the enzyme intermediate in favor of coupling) or by thermodynamic methods (an equilibrium approach that requires shifting the position of equilibrium). See Kullman, Enzymatic Peptide Synthesis (CRC Press 1987).

Four papers describe methods involving protease enzymes for the kinetic and thermodynamic syntheses of the peptide bond between α-amino acids or α-amino acid derivatives (acyl donors) and amines (synthetic non-α-amino -acid nucleophiles).

One such paper is Fischer et al., "Papain-catalyzed Peptide Synthesis in Organic Solvent Systems with Extreme Low Water Content," Peptide Chemistry 413 (1987), which discloses a kinetic method of coupling Boc-Tyr-Gly-$OCH_2CONH_2$ (acyl donor) to pentyl amine (a synthetic non-α-amino-acid nucleophile) using papain to form Boc-Tyr-Gly-NHpentyl. The method does not appear to be a true enzyme-catalyzed reaction as the use

of papain results in only a 13% improvement in yield over the same reaction without the use of papain.

Barbas et al., in J. Chem. Soc., Chem. Comm. 533 (1987), describe another kinetic method in the papain-catalyzed coupling of Z-Gly-OEt (acyl donor) to ε-aminocaproic acid methyl ester (a synthetic non-α-amino - acid nucleophile) to prepare Z-Gly-ε-ACA-OMe. This method is impractical as the reaction requires a large excess (100%) of expensive nucleophile and results in a yield of coupled product of only 31%, based on the amount of nucleophile. Another deficiency of such kinetic methods, which was evident from both the Fischer et al. and Barbas et al. reports, is the required use of high pH, which often leads to racemization of the peptide, thus rendering the product useless for its intended purpose.

Cerovsky et al., in 52 Coll. Czech. Chem. Comm. 2309 (1987), reported studies where papain-catalyzed thermodynamic methods were used to couple Z-Cys(Sbenzyl) (acyl donor) to a series of non-α-amino-acid nucleophiles of different base strengths. Although the authors report good yields with weakly basic amines (e.g., phenyl hydrazine, 90% yield, $pK_B$=8.79; aniline, 77% yield, $pK_B$=9.37), the use of strongly basic amine nucleophiles resulted in extremely poor yields (e.g., benzylamine, 19% yield, $pk_B$=4.67; cyclohexylamine, 0% yield, $pK_B$=3.34). They concluded that "the amide bond formation requires conjugation of the aromatic system with the amino group of the given amine." Since nucleophiles of the general structure II are not conjugated with aromatic systems and are strongly basic amines ($pK_B$=3-4), the results suggest that they would not work in the present application.

Cerovsky et al., in 49 Coll. Czech. Chem. Comm. 2557 (1984), also disclose a thermodynamic method of papain-catalyzed coupling of N-substituted amino acids with stoichiometric amounts of aniline or phenylhydrazine nucleophiles. The method gave poor yields (average 56% ± 23%), required long reaction times (24 hrs), and gave very poor volumetric productivity.

U.S. Patent No. 4,889,869 discloses a wide variety of methods of synthesizing peptide bonds in renin inhibitors of the type noted above, including enzymatic syntheses that supposedly use the proteases thermolysin, carboxypeptidase Y, chymotrypsin, trypsin, pepsin and papain. However, it is apparent that no such actual syntheses were conducted since none of the named proteases, with the exception of papain, actually work in an enzymatic synthesis reaction to form the peptide bond of concern.

The present invention overcomes the deficiencies of the aforementioned prior art methods of synthesizing renin inhibitors, and provides a fast and economical method that gives near-quantitative yields with no secondary reactions and no racemization.

## Summary of the Invention

In the broadest aspect of the present invention, it has been found that peptide renin inhibitors of structure III shown above may be simply and cheaply synthesized by coupling a component peptide acyl donor of structure I with a component nucleophile of structure II in the presence of a class of proteases that, when a preparation of the protease is present in the form of a supported catalyst containing 4 to 20 wt% water at pH 6.5 to 7.5 in a water-saturated ethyl acetate solution at 25°C to 60°C containing 100 to 500 mM of both the peptide acyl donor N-acetyl-L-phenylalanyl-L-alanine methyl ester (Ac-Phe-Ala-OMe) and the nucleophile (3S,2R)-cyclohexylnorstatine isopropyl ester (Norst-$OPr^i$), catalyzes a coupling of the carboxyl terminus of the peptide acyl donor and the amino group of the nucleophile with

(a) a normalized initial reaction velocity of ≧1.0 millimole of peptide coupled per liter of solution per hour per weight percent enzyme;

(b) a regioselectivity of ≧20:1 for coupling relative to cleavage of the phenylalanyl-alanine peptide bond; and

(c) a diastereoselectivity of ≧20:1 in the formation of Ac-L-Phe-L-Ala-Norst-$Opr^i$ to the formation of Ac-L-Phe-D-Ala-Norst-$Opr^i$.

The terms "normalized initial reaction velocity," "regioselectivity" and "diastereoselectivity" are defined in the Detailed Description of the Invention which follows.

## Detailed Description of the Invention

Reference is made herein to Tables 1 and 2. Table 1 is a master list of all compounds described, using the same Roman numerals noted in the Background of the Invention, that is, I for acyl donors, II for nucleophiles, and III for tripeptide renin inhibitors. Table 2 is a summary of the structures of the coupling components used and renin inhibitors synthesized in each Example. All configurations are (S) unless designated (R).

According to the present invention there is provided an enzymatic method of synthesizing peptide renin inhibitors of the general structure III noted above comprising reacting a peptide acyl donor of the structure I with a nucleophile of the structure II in the presence of a preparation of a protease that, in the reference coupling reaction noted, exhibits the coupling reaction velocity, regiospecificity and diastereoselectivity set forth above in the Summary of the Invention, where the substituents are defined as follows:

R is hydrogen, alkyl, cycloalkyl, aryl or aralkyl;

$R_1$ is ROCONH-, ROCOO-, $ROCOCH_2$-, RNHCONH-, RNHCOO-, $RNHCOCH_2$-, $RSO_2NH$-, or $RSO_2CH_2$-;

$R_2$, $R_4$ and $R_6$ are each independently alkyl, cycloalkyl, aryl or aralkyl;

$R_3$ is hydrogen, alkyl, cycloalkyl, 4-imidazoyl, -OH, -SR, $-(CH_2)_n$-SR, or $-(CH_2)_n-NH_2$ where n is an integer from 1 to 6;

$R_6$ is $-COOR_6$, $-CHOHR_6$, $-CH_2CONHCH_2R_6$, or $-CH_2CHRCONHR_6$;

"alkyl" is substituted or unsubstituted straight or branched chain carbon groups of 1 to 20 carbon atoms, which may contain heteroatoms;

"cycloalkyl" is a ring structure containing 3 to 7 carbon atoms, which may contain bridged structures or heteroatoms in the ring where the heteroatom is selected from O, N and S, or substituted cycloalkyl containing at least one substituent that may contain an atom selected from nitrogen, halogen, carbon, oxygen, phosphorous and sulfur.

"aryl" is monocyclic, bicyclic or heterocyclic aromatic groups containing 6 to 10 carbon atoms where the heteratom is selected from oxygen, nitrogen and sulfur or substituted aryl containing at least one substituent that may contain an atom selected from nitrogen, halogen, carbon, oxygen, phosphorous and sulfur; and

"aralkyl" is an alkyl group bonded to an aryl group.

The reference coupling reaction comprises the enzymatic coupling of equimolar amounts of the component dipeptide ester acyl donor and nucleophile under standard conditions. More specifically, 250 mM of the peptide acyl donor Ac-Phe-Ala-OMe (structure Iv in Table 1 below) in a water-saturated ethyl acetate solution is reacted at 50°C with the same amount (250 mM) of the nucleophile Norst-OPr$^i$ (structure IIa in Table 1 below) in the presence of 5 wt% of a supported catalyst comprising the protease preparation. The supported catalyst is prepared by combining 3 parts by weight 0.1 M pH 7.0 aqueous phosphate buffer solution containing 1.6 wt% protease preparation and 0.6 wt% cysteine (as an adjuvant) with 1 part by weight porous calcined diatomaceous earth, and allowing water to evaporate to 7 wt% water content.

The preferred protease preparations useful in this invention exhibit three principal characteristics:

(1) They catalyze the coupling of the acyl donor acids with amino donors at a sufficiently rapid rate. This characteristic is reflected in the normalized initial reaction velocity for coupling, defined as millimoles of coupling product produced per liter of solution per hour per weight percent enzyme. As noted above, a useful reference reaction is the coupling of Ac-Phe -Ala-OMe (Iv) and Norst-OPr$^i$ (IIa) to form N-acetyl-L-phenylalanyl -L-alanyl-(3S,2R)-cyclohexylnorstatine isopropyl ester (Ac-Phe-Ala-Norst-OPr$^i$) (IIIv). Protease preparations for which the reference reaction velocity is less than 1 millimole of coupling product produced per liter of solution per hour per weight percent enzyme are not deemed useful in the present invention.

(2) They catalyze the coupling of the acyl donors with amino donors with sufficient regioselectivity. "Regioselectivity" is reflected in and is defined as the ratio of the reaction velocity for coupling relative to the reaction velocity for internal amide bond cleavage. As noted above, a useful reference is the ratio of the normalized initial reaction velocities of the coupling and the cleavage reaction that leads to the production of N-acetyl-L-phenylalanine in the acid form. Protease preparations for which the ratio of these two reference reactions is at least 100:1 are preferred because they result in the production of a renin inhibitor coupling product that is at least 99% chemically pure, a purity which is generally regarded to be acceptable in pharmaceutical applications; those for which the same ratio is at least 20:1 are nevertheless deemed useful in the present invention because they result in the production of renin inhibitors that can be purified to 99% chemically pure.

(3) They catalyze the coupling of the acyl donors with amino donors with sufficient diastereoselectivity . "Diastereoselectivity" is reflected in and is defined as the ratio of the normalized initial reaction velocities for formation of the L-isomer in preference to the D-isomer for coupling with an acyl donor where the $R_3$-bearing site in the acyl donor of structure I above has undergone epimerization. In the same reference reaction noted above, the competing reactions are the couplings of N-acetyl-L-phenylalanyl-L-alanine and its epimer N-acetyl-L-phenylalanyl-D-alanine with (3S,2R)-cyclohexylnorstatine isopropyl ester. Protease preparations for which the ratio of the coupling rate of the L-L isomer to the coupling rate of the L-D isomer is at least 100:1 are preferred, but those for which the ratio is at least 20:1 are nevertheless deemed useful for the same reasons mentioned above in paragraph (2).

A number of preferred protease preparations falling within the above functional parameters are those of sulfhydryl proteases. Examples of specific protease preparations found useful in the present invention include papain, chymopapain, ficin, and bromelain. The form in which such protease preparations may be used includes a powder, a solution, immobilized on a support, an enzyme-containing cellular extract, and enzyme-containing cells. All of these forms may be dissolved or suspended in water, an organic solvent, or a mixture of the two. When in the form of enzyme-containing cells, the cells may be whole or fragmented.

Immobilization of the protease preparation on an inert support such as bleached or unbleached or calcined diatomaceous earth, polydextran, polyacrylamide, polystyrene, polyacrylic acid ester, silica gel or porous glass beads is also advantageous in conducting the coupling process of the present invention. Preferred commercially available forms of calcined diatomaceous earth are those made and sold by Johns Manville of Denver, Colorado under the "Celite" series. Preferred commercially available forms of polyacrylic acid ester are those made and sold under the names "XAD-7" and "XAD-8" by Rohm & Haas of Philadelphia, Pennsylvania. A preferred commercially available form of polydextran is made and sold under the name "Sephadex" by the Swedish company Pharmacia. Preferred commercially available forms of polyacrylamide are those made and sold as the "Biogel P" series by Bio-Rad Laboratories of Richmond, California.

Water content of such immobilized enzymatic catalysts should be from 0.5 to 10.0 wt%, preferably 7.0 wt%. Enzyme loading, based upon weight of the support, should be from 2.0 to 20 wt%, preferably 5.0 wt%. Enhanced reaction times and yields may be obtained with the addition of adjuvants such as cystamine, cysteine, beta-mercaptoethanol, hydrogen sulfide, and dithiothreitol. Such adjuvants may be added to the immobilized enzyme or to the solution or suspension enzyme in an amount from 1.0 to 5.0 wt%, preferably 2.0 wt%. Generally speaking, increasing the concentration of reactants in the coupling reaction of the present invention increases the rate of renin inhibitor peptide production.

Reaction rates also increase with increasing temperature (from 20°C to 60°C), and with an increase in catalyst concentration and specific activity of catalysts, catalyst specific activity being specified in terms of number of units of activity per gram. A preferred range of specific activity of catalyst is 75 to 500 units per gram, while a preferred range of catalyst concentration is 100 to 500 mg/ml. A unit of activity is defined as the amount of enzyme preparation needed to hydrolyze the carboxyl terminus of the substrate N-acetyl-L-phenylalanyl-L-alanine methyl ester at a rate equal to 1 micromole of methyl ester hydrolyzed per liter of solution per minute at 25°C in a reaction solution at pH 6.5 containing 5 vol% acetonitrile in water. The protease catalysts of the present invention appear to be reusable up to a minimum of five times.

The pH of the reaction solution may be from about 6.5 to 7.5, preferably essentially neutral.

In an especially preferred embodiment, peptide acyl donor acids that participate in the coupling reaction are formed in situ by starting with the ester form of the acyl donor, as exemplified in the Examples.

Table 1

| Structure No. | $R_1$ | $R_2$ | $R_3$ | $R_4$ | $R_5$ | R | Abbreviations |
|---|---|---|---|---|---|---|---|
| Ia | $(CH_3)_3COCONH-$ | -phenyl | -H | | | $-CH_3$ | Boc-Phe-Ala-OMe |
| Ic | do | do | -H (R) | | | do | Boc-Phe-D-Ala-OMe |
| Id | do | do | $-SCH_2C_6H_5$ | | | do | Boc-Phe-(SBzl)Cys-OMe |
| Ie | do | $-C_6H_4OCH_3$ | $-SCH_3$ | | | do | Boc-(OMe)Tyr-(SMe)Cys-OMe |
| If | do | -phenyl | -imidazoyl | | | do | Boc-Phe-His-OMe |
| Ig | do | do | $-CH(CH_3)_2$ | | | do | Boc-Phe-Leu-OMe |
| Ih | do | do | $-CH_2SCH_3$ | | | do | Boc-Phe-Met-OMe |
| Ii | do | do | $-CH_2SCH_3$ (R) | | | do | Boc-Phe-D-Met-OMe |
| Ik | do | do | -phenyl | | | do | Boc-Phe-Phe-OMe |
| Il | do | do | -phenyl (R) | | | do | Boc-Phe-D-Phe-OMe |
| Im | N-morpholino-CONH- | do | $-SCH_2C_6H_5$ | | | do | Mor-Phe-(SBzl)Cys-OMe |
| In | do | do | $-SCH_3$ | | | do | Mor-Phe-(SMe)Cys-OMe |
| Iq | do | do | -imidazoyl | | | do | Mor-Phe-His-OMe |
| Ir | do | do | -imidazoyl (R) | | | do | Mor-Phe-D-His-OMe |
| It | do | do | $-CH(CH_3)_2$ | | | do | Mor-Phe-Leu-OMe |
| Iu | do | do | $-CH_2SCH_3$ | | | do | Mor-Phe-Met-OMe |
| Iv | $CH_3CONH-$ | do | -H | | | do | Ac-Phe-Ala-OMe |
| IIa | | | | -cyclohexyl | $-COOCH(CH_3)_2$ | | Norst-OPr$^i$ |
| IIb | | | | do | $-CH(OH)CH_2CH(CH_3)_2$ | | Diol |
| IIc | | | | -phenyl | -H | | Phe-OH |
| IId | | | | $-CH(CH_3)_2$ | do | | Leu-OH |
| IIIa | $(CH_3)_3COCONH-$ | -phenyl | -H | -cyclohexyl | $-COOCH(CH_3)_2$ | | Boc-Phe-Ala-Norst-OPr$^i$ |
| IIIc | do | -phenyl | -H (R) | do | do | | Boc-Phe-D-Ala-Norst-OPr$^i$ |
| IIId | do | do | $-SCH_2C_6H_5$ | do | do | | Boc-Phe-(SBzl)Cys-Norst-OPr$^i$ |
| IIIe | do | $-C_6H_4OCH_3$ | $-SCH_3$ | do | do | | Boc-(OMe)Tyr-(SMe)Cys-Norst-OPr$^i$ |
| IIIf | do | -phenyl | -imidazoyl | do | do | | Boc-Phe-His-Norst-OPr$^i$ |
| IIIg | do | do | $-CH(CH_3)_2$ | do | do | | Boc-Phe-Leu-Norst-OPr$^i$ |
| IIIh | do | do | $-CH_2SCH_3$ | do | do | | Boc-Phe-Met-Norst-OPr$^i$ |
| IIIi | do | do | $-CH_2SCH_3$ (R) | do | do | | Boc-Phe-D-Met-Norst-OPr$^i$ |
| IIIj | do | do | $-CH_2SCH_3$ | do | $-CH(OH)CH_2CH(CH_3)_2$ | | Boc-Phe-Met-Diol |
| IIIk | do | do | -phenyl | do | $-COOCH(CH_3)_2$ | | Boc-Phe-Phe-Norst-OPr$^i$ |
| IIIl | do | do | -phenyl (R) | do | do | | Boc-Phe-D-Phe-Norst-OPr$^i$ |

Table 1 (Cont.)

| Structure No. | $R_1$ | $R_2$ | $R_3$ | $R_4$ | $R_5$ | R | Abbreviations |
|---|---|---|---|---|---|---|---|
| IIIm | N-morpholino-CONH- | -phenyl | $-SCH_2C_6H_5$ | -cyclohexyl | $-COOCH(CH_3)_2$ | | Mor-Phe-(SBzl)Cys-Norst-OPr[i] |
| IIIn | do | do | $-SCH_3$ | do | do | | Mor-Phe-(SMe)Cys-Norst-OPr[i] |
| IIIo | do | do | do | -phenyl | -H | | Mor-Phe-(SMe)Cys-Phe-OH |
| IIIp | do | do | do | $-CH(CH_3)_2$ | do | | Mor-Phe-(SMe)Cys-Leu-OH |
| IIIq | do | do | -imidazoyl | -cyclohexyl | $-COOCH(CH_3)_2$ | | Mor-Phe-His-Norst-OPr[i] |
| IIIr | do | do | -imidazoyl (R) | do | do | | Mor-Phe-D-His-Norst-OPr[i] |
| IIIs | do | do | -imidazoyl | do | $-CH(OH)CH_2CH(CH_3)_2$ | | Mor-Phe-His-Diol |
| IIIt | do | do | $-CH(CH_3)_2$ | do | $-COOCH(CH_3)_2$ | | Mor-Phe-Leu-Norst-OPr[i] |
| IIIu | do | do | $-CH_2SCH_3$ | do | do | | Mor-Phe-Met-Norst-OPr[i] |
| IIIv | $CH_3CONH-$ | do | -H | do | do | | Ac-Phe-Ala-Norst-OPr[i] |

Table 2

| Example No. | Structure | $R_1$ | $R_2$ | $R_3$ | $R_4$ | $R_5$ | R | Abbreviations |
|---|---|---|---|---|---|---|---|---|
| 1-19,31,36, | In | N-morpholino-CONH- | -phenyl | $-SCH_3$ | | | $-CH_3$ | Mor-Phe-(SMe)Cys-OMe |
| 48,54,56, | IIa | | | | -cyclohexyl | $-COOCH(CH_3)_2$ | | Norst-OPr$^{i}$ |
| 58 | IIIn | do | do | do | do | do | | Mor-Phe-(SMe)Cys-Norst-OPr$^{i}$ |
| 20,37,60 | Ia | $(CH_3)_3COCONH-$ | do | -H | | | do | Boc-Phe-Ala-OMe |
| | IIa | | | | do | do | | Norst-OPr$^{i}$ |
| | IIIa | do | do | do | do | do | | Boc-Phe-Ala-Norst-OPr$^{i}$ |
| 21,38 | Id | do | do | $-SCH_2C_6H_5$ | | | do | Boc-Phe-(SBzl)Cys-OMe |
| | IIa | | | | do | do | | Norst-OPr$^{i}$ |
| | IIId | do | do | do | do | do | | Boc-Phe-(SBzl)Cys-Norst-OPr$^{i}$ |
| 22,39 | Ie | do | $-C_6H_4OCH_3$ | $-SCH_3$ | | | do | Boc-(OMe)Tyr-(SMe)Cys-OMe |
| | IIa | | | | do | do | | Norst-OPr$^{i}$ |
| | IIIe | do | do | do | do | do | | Boc-(OMe)Tyr-(SMe)Cys-Norst-OPr$^{i}$ |
| 23,40 | If | do | -phenyl | -imidazoyl | | | do | Boc-Phe-His-OMe |
| | IIa | | | | do | do | | Norst-OPr$^{i}$ |
| | IIIf | do | do | do | do | do | | Boc-Phe-His-Norst-OPr$^{i}$ |
| 24,41 | Ig | do | do | $-CH(CH_3)_2$ | | | do | Boc-Phe-Leu-OMe |
| | IIa | | | | do | do | | Norst-OPr$^{i}$ |
| | IIIg | do | do | do | do | do | | Boc-Phe-Leu-Norst-OPr$^{i}$ |
| 25,42 | Ih | do | do | $-CH_2SCH_3$ | | | do | Boc-Phe-Met-OMe |
| | IIb | | | | do | $-CH(OH)CH_2CH(CH_3)_2$ | | Diol |
| | IIIj | do | do | do | do | do | | Boc-Phe-Met-Diol |
| 26,43,62 | Ih | do | do | do | | | do | Boc-Phe-Met-OMe |
| | IIa | | | | do | $-COOCH(CH_3)_2$ | | Norst-OPr$^{i}$ |
| | IIIh | do | do | do | do | do | | Boc-Phe-Met-Norst-OPr$^{i}$ |
| 27,44,64 | Ik | do | do | -phenyl | | | do | Boc-Phe-OMe |
| | IIa | | | | -cyclohexyl | do | | Norst-OPr$^{i}$ |
| | IIIk | do | do | do | do | do | | Boc-Phe-Phe-Norst-OPr$^{i}$ |
| 28,45 | Im | N-morpholino-CONH- | do | $-SCH_2C_6H_5$ | | | do | Mor-Phe-(SBzl)Cys-OMe |
| | IIa | | | | do | do | | Norst-OPr$^{i}$ |
| | IIIm | do | do | do | do | do | | Mor-Phe-(SBzl)Cys-Norst-OPr$^{i}$ |

Table 2 (cont.)

| Example No. | Structure | $R_1$ | $R_2$ | $R_3$ | $R_4$ | $R_5$ | R | Abbreviations |
|---|---|---|---|---|---|---|---|---|
| 29,46 | In | N-morpholino-CONH- | -phenyl | $-SCH_3$ | | | $-CH_3$ | Mor-Phe-(SMe)Cys-OMe |
| | IId | | | | $-CH(CH_3)_2$ | -H | | Leu-OH |
| | IIIp | do | do | do | do | do | | Mor-Phe-(SMe)Cys-Leu-OH |
| 30,47 | In | do | do | do | | | do | Mor-Phe-(SMe)Cys-OMe |
| | IIc | | | | -phenyl | -H | | Phe-OH |
| | IIIo | do | do | do | do | do | | Mor-Phe-(SMe)Cys-Phe-OH |
| 32,49,66 | Iq | do | do | -imidazoyl | | | do | Mor-Phe-His-OMe |
| | IIa | | | | -cyclohexyl | $-COOCH(CH_3)_2$ | | Norst-$OPr^i$ |
| | IIIq | do | do | do | do | do | | Mor-Phe-His-Norst-$OPr^i$ |
| 33,50 | Iq | do | do | do | | | do | Mor-Phe-His-OMe |
| | IIb | | | | do | $-CH(OH)CH_2CH(CH_3)_2$ | | Diol |
| | IIIs | do | do | do | do | do | | Mor-Phe-His-Diol |
| 34,51 | It | do | do | $-CH(CH_3)_2$ | | | do | Mor-Phe-Leu-OMe |
| | IIa | | | | do | $-COOCH(CH_3)_2$ | | Norst-$OPr^i$ |
| | IIIt | do | do | do | do | do | | Mor-Phe-Leu-Norst-$OPr^i$ |
| 35,52 | Iu | do | do | $-CH_2SCH_3$ | | | do | Mor-Phe-Met-OMe |
| | IIa | | | | do | do | | Norst-$OPr^i$ |
| | IIIu | do | do | do | do | do | | Mor-Phe-Met-Norst-$OPr^i$ |
| 61 | Ic | $-CH_3)_3COCONH-$ | do | -H (R) | | | do | Boc-Phe-D-Ala-OMe |
| | IIa | | | | do | do | | Norst-$OPr^i$ |
| | IIIc | do | do | do | do | do | | Boc-Phe-D-Ala-Norst-$OPr^i$ |
| 63 | Ii | do | do | $-CH_2SCH_3$ (R) | | | do | Boc-Phe-D-Met-OMe |
| | IIa | | | | do | do | | Norst-$OPr^i$ |
| | IIIi | do | do | do | do | do | | Boc-Phe-D-Met-Norst-$OPr^i$ |
| 65 | Il | do | do | -phenyl (R) | | | do | Boc-Phe-D-Phe-OMe |
| | IIa | | | | do | do | | Norst-$OPr^i$ |
| | IIIl | do | do | do | do | do | | Boc-Phe-D-Phe-Norst-$OPr^i$ |
| 67 | Ir | N-morpholino-CONH- | do | -imidazoyl (R) | | | do | Mor-Phe-D-His-OMe |
| | IIa | | | | do | do | | Norst-$OPr^i$ |
| | IIIr | do | do | do | do | do | | Mor-Phe-D-His-Norst-$OPr^i$ |

Table 2 (cont.)

| Example No. | Structure | $R_1$ | $R_2$ | $R_3$ | $R_4$ | $R_5$ | R | Abbreviations |
|---|---|---|---|---|---|---|---|---|
| 53,55, | Iv | $CH_3COHN-$ | -phenyl | -H | | | $-CH_3$ | Ac-Phe-Ala-OME |
| 57,59 | IIa | | | | -cyclohexyl | $-COOCH(CH_3)_2$ | | Norst-OPr$^i$ |
| | IIIv | do | do | do | do | | | Ac-Phe-Ala-Norst-OPr$^i$ |

Example 1

This Example illustrates the process of the present invention in terms of the reference coupling reaction using an acyl donor in the ester form. The enzyme catalyst was prepared by dissolving 250 mg papain preparation in 16 ml of 0.1 M phosphate buffer at pH 7.0, along with 100 mg of L-cysteine, to form a catalyst solution containing 1.6 wt% of the protease preparation and 0.6 wt% of the adjuvant L-cysteine. The pH was readjusted to 7.0 with 0.1 M NaOH. Three parts by weight of this solution was combined with one part by weight (5 g) of porous calcined diatomaceous earth (Celite 649) to form a slurry, and transferred to a watch glass. The water in the preparation was allowed to evaporate overnight to form a 5 wt% loaded enzyme catalyst. The dried cake was gently broken up to give a free-flowing, granular preparation. Residual water was measured to be 7 wt% by determining the weight loss of a known amount of preparation upon heating to 150°C under vacuum. Five ml of a solution of 250 mM of the peptide acyl donor Mor-Phe -(SMe)Cys-OMe (having the structure In) and 250 mM of the nucleophile (3S, 2R)-cycohexylnorstatine isopropyl ester (having the structure IIa) in water saturated ethyl acetate (EtOAc) was added to 1.25 g of catalyst (to form a final catalyst concentration of 250 mg/ml), and the mixture stirred at 50°C to produce a normalized initial reaction velocity or rate of 96 mM/hr/wt% enzyme, the reaction proceeding to 100% conversion of the tripeptide renin inhibitor IIIn in 7 hours as indicated by HPLC analysis.

Examples 2-7

Example 1 was repeated with the exception that the catalyst was immobilized on various inert supports. Supports tested were Celite 577 (unbleached diatomaceous earth); Celite 503 (bleached diatomaceous earth); Celite 649 (calcined diatomaceous earth, 50/100 mesh); Celite 648 (calcined diatomaceous earth, 30/50 mesh), XAD-8 (polyacrylic acid ester), and silica gel. Normalized initial reaction rates and times to 50% conversion are reported in Table 3.

Table 3

| Ex. No. | Support | Normalized Initial Rate (mM/hr/wt% enz) | Time for 50% Conversion (hrs) |
|---|---|---|---|
| 2 | Celite 503 | 48 | 2 |
| 3 | Celite 577 | 46 | 2 |
| 4 | Celite 648 | 49 | 2 |
| 5 | Celite 649 | 44 | 2.1 |
| 6 | XAD-8 | 13 | 8 |
| 7 | Silica | 28 | 4 |

Examples 8-11

Example 1 was repeated with the exception that the loading of the catalyst was varied. Results are shown in Table 4.

Table 4

| Ex. No. | Enzyme Loading (wt%) | Initial Rate (mM/hr) | Normalized Initial Rate (mM/hr/wt% enz) |
|---|---|---|---|
| 8 | 2.5 | 60 | 24 |
| 9 | 5 | 130 | 26 |
| 10 | 10 | 110 | 11 |
| 11 | 20 | 90 | 4.5 |

The optimum loading was determined to be 5 wt%, with 10% giving nearly the same activity and 20% slightly lower activity. This may indicate that at 5% loading a single layer of enzyme covers the support, as higher loadings do not seem to increase the amount of available enzyme.

Examples 12-14

Example 1 was repeated with the exception that papain catalyst was prepared with and without the adjuvant cysteine in the reaction solution at enzyme loadings of 5 and 10 wt%. Catalyst concentration was 500 mg/ml and the reaction was conducted at 50°C. The results, shown in Table 5, indicate that at both enzyme loadings the presence of cysteine in solution enhanced catalytic activity.

Table 5

| Ex. No. | Enzyme Loading (wt%) | Cysteine (wt%) | Normalized Initial Rate (mM/hr/wt% enz) |
|---|---|---|---|
| 12 | 5 | 2 | 82 |
| 13 | 10 | 0 | 62 |
| 14 | 10 | 2 | 85 |

Example 15

Example 1 was repeated with the exception that the reaction was conducted in a heated column reactor while recirculating the reactants through the column, which was packed with 37 g of the enzyme catalyst. Substantially the same results were achieved.

Examples 16-19

Example 1 was substantially repeated with various enzyme preparation catalysts. The results are shown in Table 6.

Table 6

| Ex. No. | Enzyme | Normalized Initial Rate (mM/hr/wt% enz) | Time For Conversion (hrs) | | Yield (%) |
|---|---|---|---|---|---|
| | | | 50% | Max | |
| 16 | papain | 96 | 1.5 | 7 | 100 |
| 17 | ficin | 30 | 8 | 72 | 75 |
| 18 | bromelain | 3.8 | 48 | 120 | 100 |
| 19 | chymopapain | 110 | 1.2 | 5 | 100 |

Examples 20-35

These Examples illustrate that a wide range of tripeptide renin inhibitor structures is amenable to synthesis by the process of the present invention. Solutions of acyl donors in the form of dipeptide methyl esters of general structure I and nucleophiles of general structure II (250 mM each) in the same solvent and catalyst system as in Example 1 were stirred at 50°C. The results are summarized in Table 7.

Table 7

| Ex. No. | Structures | | | Normalized Initial Rate (mM/hr/wt% enz) | Time For Conversion (hrs) | | Yield (%) |
|---|---|---|---|---|---|---|---|
| | | | | | 50% | Max | |
| 20 | Ia | IIa | IIIa | 120 | 0.9 | 6 | 100 |
| 21 | Id | IIa | IIId | 120 | 1.3 | 7.5 | 100 |
| 22 | Ie | IIa | IIIe | 13 | 60 | 72 | 70 |
| 23 | If | IIa | IIIf | 8.7 | - | 72 | 20 |
| 24 | Ig | IIa | IIIg | 7.2 | 45 | 72 | 62 |
| 25 | Ih | IIb | IIIj | 18 | - | 72 | 14 |
| 26 | Ih | IIa | IIIh | 130 | 1.1 | 7.5 | 100 |
| 27 | Ik | IIa | IIIk | 48 | 2.5 | 18 | 84 |
| 28 | Im | IIa | IIIm | 120 | 1.2 | 7 | 100 |
| 29 | In | IId | IIIp | 24 | 60 | 72 | 64 |
| 30 | In | IIc | IIIo | 51 | 2.5 | 12 | 95 |
| 31 | In | IIa | IIIn | 96 | 1.5 | 7 | 100 |
| 32 | Iq | IIa | IIIq | 7.7 | 13.8 | 48 | 72 |
| 33 | Iq | IIb | IIIs | 3.6 | - | 28 | 10 |
| 34 | It | IIa | IIIt | 8.6 | 25 | 46 | 95 |
| 35 | Iu | IIa | IIIu | 120 | 1 | 3 | 100 |

Example 36

To illustrate that in situ production of the acyl donor acid provides unexpectedly rapid coupling, syntheses of compound IIIn were performed using compound In in both the methyl ester form (R = $-CH_3$) and in the acid form (R = -H) as acyl donor starting materials and structure IIa as the nucleophile. Both reactions contained 250 mM starting materials in EtOAc and 250 mg/ml immobilized papain. When the ester was used as starting material, an initial rate of 100 mM/hr was achieved, which rate was sustained for the approximately 4 hours it took to complete the reaction. By contrast, the initial rate of tripeptide synthesis for the acid form was 28 mM/hr, which rate was also substantially sustained for 4 hours.

Examples 37-59

These Examples illustrate the high degree of specificity for the coupling between the carboxyl terminus of the dipeptide ester and the amino group of the nucleophile that is provided by the method of the present invention. A competing reaction during the coupling reaction is cleavage of the peptide bond of the acyl donor of structure I. Regioselectivity was defined in the reference coupling reaction of Ac-Phe-Ala-OMe and Norst-Opr$^i$ as the ratio of normalized initial rate of tripeptide synthesis (or coupling) to the normalized initial rate of cleavage of the phenylalanyl-alanine peptide bond, evidenced by cleavage product production. When no cleavage product was detected, a rate of 0.8 μM/hr/wt% enzyme was used as an upper limit for the calculation. To demonstrate regioselectivity, syntheses of various tripeptides with the same reactants in substantially the same manner as in Example 1 were analyzed for the presence of a carboxylic acid bearing the substituents $R_1$ and $R_2$ as an indicator of this type of internal cleavage. Control experiments indicate that rates as slow as 0.8 μM/hr/wt% enzyme would be detected after 24 hours. The synthesis reactions were examined after 24 hours for the presence of cleavage product. The results of these analyses are shown in Table 8. The lowest measured regioselectivity was 4000 which corresponds to the production of coupling product with a chemical purity of 99.97%. Regioselectivities as low as 20, corresponding to the production of coupling product with a chemical purity of 95%, are also useful in the present invention because such mixtures can generally be purified by conventional methods to the 99.90% chemical purities required for pharmaceuticals for human use.

Table 8

| Ex. No. | Structures | | | Enzyme | Normalized Initial Rate (mM/hr/wt% enz) | Yield (%) | Regio-Selectivity |
|---|---|---|---|---|---|---|---|
| 37 | Ia | IIa | IIIa | papain | 120 | 100 | 144000 |
| 38 | Id | IIa | IIId | papain | 120 | 100 | 144000 |
| 39 | Ie | IIa | IIIe | papain | 13 | 70 | 16000 |
| 40 | If | IIa | IIIf | papain | 8.7 | 20 | 10000 |
| 41 | Ig | IIa | IIIg | papain | 7.2 | 62 | 9000 |
| 42 | Ih | IIb | IIIj | papain | 18 | 14 | 22000 |
| 43 | Ih | IIa | IIIh | papain | 130 | 100 | 156000 |
| 44 | Ik | IIa | IIIk | papain | 48 | 84 | 58000 |
| 45 | Im | IIa | IIIm | papain | 120 | 100 | 144000 |
| 46 | In | IId | IIIp | papain | 24 | 64 | 29000 |
| 47 | In | IIc | IIIo | papain | 51 | 95 | 61000 |
| 48 | In | IIa | IIIn | papain | 96 | 100 | 115000 |
| 49 | Iq | IIa | IIIq | papain | 7.7 | 72 | 9000 |
| 50 | Iq | IIb | IIIs | papain | 3.6 | 10 | 4000 |
| 51 | It | IIa | IIIt | papain | 8.6 | 95 | 10000 |
| 52 | Iu | IIa | IIIu | papain | 120 | 100 | 144000 |
| 53 | Iv | IIa | IIIv | papain | 130 | 100 | 160000 |
| 54 | In | IIa | IIIn | ficin | 30 | 75 | 36000 |
| 55 | Iv | IIa | IIIv | ficin | 88 | 100 | 110000 |
| 56 | In | IIa | IIIn | bromelain | 3.8 | 100 | 5000 |
| 57 | Iv | IIa | IIIv | bromelain | 9.6 | 100 | 12000 |
| 58 | In | IIa | IIIn | chymopapain | 110 | 100 | 132000 |
| 59 | Iv | IIa | IIIv | chymopapain | 140 | 100 | 170000 |

Examples 60-67

These Examples demonstrate that the method of the present invention is diastereoselective, that is, that it results in the production of a tripeptide strongly favoring the (L) configuration at the $R_3$ substituent -bearing position. Diastereoselectivity was defined as the ratio of the normalized initial rate of synthesis of the (L,L)-isomer to that of synthesis of the (L,D)-isomer. If no synthesis was detected, the limit value of 0.8 μM/hr/wt% enzyme was used. Reactants and reaction conditions were essentially the same as for Example 1. The diastereoselectivity of the coupling method was demonstrated by the papain-catalyzed synthesis of epimers of four tripeptides: IIIa and IIIh; IIIi and IIIr; IIIi and IIIj; and IIIk and IIIl. In all cases, a D-amino acid (R-configuration) at the $R_3$ substituent-bearing amino acid position prevented coupling (as well as hydrolysis of the dipeptide ester). A D-amino acid residue (R-configuration) at the $R_1$ substituent-bearing amino acid position slowed, but did not prevent, coupling. The results are shown in Table 9.

Table 9

| Ex. No. | Structures | | | Normalized Initial Rate (mM/hr/wt% enz) | Diastereo-selectivity |
|---|---|---|---|---|---|
| 60 | Ia | IIa | IIIa | 120 | 150000 |
| 61 | Ic | IIa | IIIc | <0.0008 | |
| 62 | Ih | IIa | IIIh | 130 | 162500 |
| 63 | Ii | IIa | IIIi | <0.0008 | |
| 64 | Ik | IIa | IIIk | 48 | 60000 |
| 65 | Il | IIa | IIIl | <0.0008 | |
| 66 | Iq | IIa | IIIq | 7.7 | 10000 |
| 67 | Ir | IIa | IIIr | <0.0008 | |

The terms and expressions which have been employed in the foregoing specification are used therein as terms of description and not of limitation, and there is no intention, in the use of such terms and expressions, of excluding equivalents of the features shown and described or portions thereof, it being recognized that the scope of the invention is defined and limited only by the claims which follow.

## Claims

1. A method of synthesizing peptide renin inhibitors of the structure

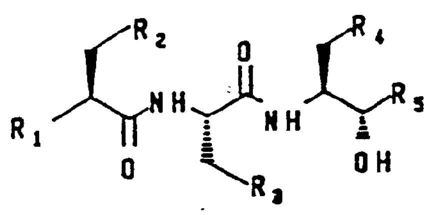

comprising reacting a peptide acyl donor of the structure

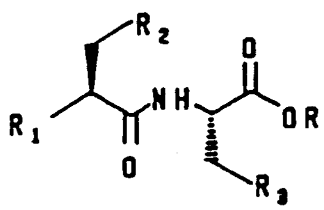

with a nucleophile of the structure

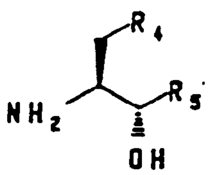

characterized in that the reaction is conducted in the presence of a protease preparation that, when present in the form of a supported catalyst containing 4 to 20 wt% water at pH 6.5 to 7.5 in a water-saturated ethyl acetate solution at 25° to 60°C containing 100 to 500 mM of both the peptide acyl donor Ac-Phe-Ala-OMe and the nucleophile Norst-OPr$^i$, catalyzes a coupling of the carboxyl terminus of said peptide acyl donor and the amino group of said nucleophile with

(a) an initial reaction velocity of ≧1.0 millimole of dipeptide coupled per liter of solution per hour per weight percent enzyme preparation;

(b) a regioselectivity ≧20:1 for coupling relative to cleavage of the phenylalanylalanine peptide bond; and

(c) a diastereoselectivity of ≧20:1 in the formation of Ac-L-Phe-L-Ala-Norst-OPr$^i$ where

R is hydrogen, alkyl, cycloalkyl, aryl or aralkyl;

$R_1$ is ROCONH-, ROCOO-, $ROCOCH_2$-, RNHCONH-, RNHCOO-, $RNHCOCH_2$-, $RSO_2NH$-, or $RSO_2CH_2$-;

$R_2$, $R_4$ and $R_6$ are each independently alkyl, cycloalkyl, aryl or aralkyl;

$R_3$ is hydrogen, alkyl, cycloalkyl, 4-imidazoyl, -OH, -SR, $-(CH_2)_n$-SR, or $-(CH_2)_n-NH_2$ where n is an integer from 1 to 6;

$R_5$ is $-COOR_6$, $-CHOHR_6$, $-CH_2CONHCH_2R_6$, or $-CH_2CHRCONHR_6$;

alkyl is substituted or unsubstituted or heteroatom-containing straight or branched chain carbon groups of 1 to 20 carbon atoms;

cycloalkyl is a ring structure containing 3 to 7 carbon atoms, which may contain bridged structures or heteroatoms in the ring, or substituted cycloalkyl;

aryl is a monocyclic, bicyclic or heterocyclic aromatic group containing 6 to 10 carbon atoms, or substituted aryl; and

aralkyl is an alkyl group bonded to an aryl group.

2. The method of claim 1 wherein said protease preparation is a sulfhydryl protease.

3. The method of claim 1 wherein said protease preparation is selected from papain, chymopapain, ficin and bromelain.

4. A method of synthesizing peptide renin inhibitors of the structure

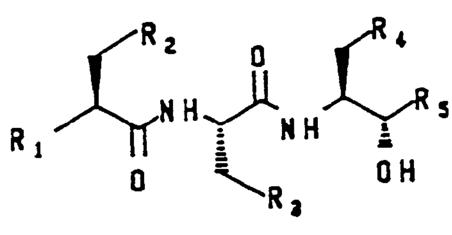

comprising reacting a peptide acyl donor of the structure

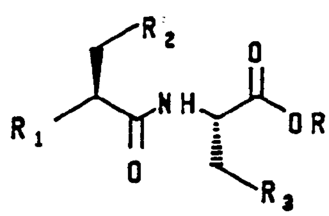

with a nucleophile of the structure

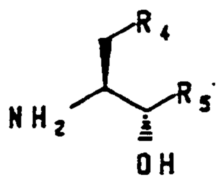

in the presence of a protease preparation comprising at least one of the group consisting essentially of papain, chymopapain, ficin and bromelain where

R is hydrogen, alkyl, cycloalkyl, aryl or aralkyl;

$R_1$ is ROCONH-, ROCOO-, ROCOCH$_2$-, RNHCONH-, RNHCOO-, RNHCOCH$_2$-, RSO$_2$NH-, RSO$_2$O-, or RSO$_2$CH$_2$-;

$R_2$, $R_4$ and $R_6$ are each independently alkyl, cycloalkyl, aryl or aralkyl;

$R_3$ is hydrogen, alkyl, cycloalkyl, 4-imidazoyl, -OH, -SR, -(CH$_2$)$_n$-SR, or -(CH$_2$)$_n$-NH$_2$ where n is an integer from 1 to 6;

$R_5$ is -COOR$_6$, -CHOHR$_6$, -CH$_2$CONHCH$_2$R$_6$, or -CH$_2$CHRCONHR$_6$;

alkyl is substituted or unsubstituted or heteroatom-containing straight or branched chain carbon groups of 1 to 20 carbon atoms;

cycloalkyl is a ring structure containing 3 to 7 carbon atoms, which may contain bridged structures or heteroatoms in the ring, or substituted cycloalkyl;

aryl is a monocyclic, bicyclic or heterocyclic aromatic group containing 6 to 10 carbon atoms, or substituted aryl; and

aralkyl is an alkyl group bonded to an aryl group.

5. The method of claim 1 or 4 wherein said protease preparation is in a form selected from a powder, a solution, immobilized on a support, an enzyme-containing cellular extract, and enzyme-containing cells.

6. The method of claim 5 wherein said protease preparation is immobilized on a support consisting essentially of porous calcined diatomaceous earth.

7. The method of claim 6 wherein said immobilized protease preparation is present in an amount of from 2 to 20 wt%, based upon weight of the support.

8. The method of claim 6 wherein said immobilized protease preparation contains 0.5 to 10 wt% water.

9. The method of claim 6 wherein said immobilized protease preparation contains an adjuvant selected from the group consisting essentially of cysteine, cystamine, beta-mercaptoethanol, hydrogen sulfide, and dithiothreitol.

10. The method of claim 5 wherein the solvent for said solution of said protease preparation is selected from water, an organic solvent, and a mixture of water and an organic solvent.

11. The method of claim 5 wherein said enzyme-containing cells are whole cells.

12. The method of claim 5 wherein said enzyme-containing cells are partial cells.